# EUROPEAN PATENT SPECIFICATION

(11) **EP 3 578 192 B1**
(45) Date of publication and mention of the grant of the patent: **01.12.2021**
(21) Application number: 17895075.4
(22) Date of filing: 31.01.2017
(51) Int. Cl.: A61K 38/03, C07K 7/06, C07K 7/08, A01N 1/02, A61D 19/02, A61K 38/08

(54) **POLYPEPTIDE SPERM STABILISER FOR SEMEN USED IN ARTIFICIAL INSEMINATION**
POLYPEPTIDSPERMIENSTABILISATOR FÜR SPERMA VERVENDET ZUR KÜNSTLICHEN BESAMUNG
STABILISATEUR SPERMATIQUE POLYPEPTIDIQUE POUR SPERME UTILE DANS L'INSÉMINATION ARTIFICIELLE

(43) Date of publication of application: 11.12.2019
(73) Proprietor: Universidad De La Frontera, Temuco 4811230 (CL); Sociedad Agrícola Y Ganadera Pehuén Ltda., Victoria, Araucanía 4720769 (CL)
(72) Inventor: ROMERO MEJÍA, Fernando Gonzalo, Temuco 4811230 (CL); JOFRÉ FERNÁNDEZ, Ignacio Andrés, Temuco 4811230 (CL); DE MIRANDA, Antonio, 05590-130 San Pablo (BR); REYNE HEISE, Sergio Andrés, Victoria Araucanía 4720769 (CL)
(74) Representative: Clarke Modet & Co.
(86) International application number: PCT/IB2017/050516
(87) International publication number: WO 2018/142184

(56) References cited:
- WO-A1-2009/043128
- WO-A1-2010/025548
- WO-A1-2010/025548
- US-A1- 2002 182 580
- US-A1- 2011 028 395
- CAGLIARI, C. I. et al.: "Action of Bauhinia bauhinioides synthetic peptides on serine proteinases", Biochemical and biophysical research communications, vol. 311, no. 1, 7 November 2003 (2003-11-07), pages 241-245, XP004465129,
- MANZANO, P. F. et al.: "Antioxidant activity of Bios-p peptide analogue in HEK293T cells and three-dimensional structure prediction", Advances in Bioscience and Biotechnology, vol. 4, no. 1, 2013, pages 55-61, XP055534796,
- SCHULZE, M. et al.: "Liquid storage of boar semen: Current and future perspectives on the use of cationic antimicrobial peptides to replace antibiotics in semen extenders", Theriogenology, vol. 85, no. 1, 2016, pages 39-46, XP029322809,
- MANJUNATH, P. et al.: "New insights into the understanding of the mechanism of sperm protection by extender components", Anim Reprod, vol. 9, no. 4, 30 September 2012 (2012-09-30), pages 809-815, XP055534823,
- REDDY, K. V. R. et al.: "Antimicrobial peptides: premises and promises", International journal of antimicrobial agents, vol. 24, no. 6, December 2004 (2004-12), pages 536-547, XP004650716,

### SEQUENCE LISTING

<110> UNIVERSIDAD DE LA FRONTERA SOCIEDAD AGRICOLA Y GANADERA PEHUEN LTDA
<120> POLYPEPTIDE SPERM STABILISER FOR SEMEN USED IN ARTIFICIAL INSEMINATION
<130> 2019/33100
<150> PCT/IB2017/050516
   <151> 0036-07-09
<160> 1
<170> BiSSAP 1.3.6
<210> 1
   <211> 10
   <212> PRT
   <213> Artificial Sequence
<220>
   <223> FB-sp spermatic stabiliser
<400> 1
   Arg Pro Gly Leu Pro Val Phe Ser Pro Leu
   1               5                   10

## Description

### FIELD OF THE INVENTION

The present invention describes a polypeptide molecule having pharmacodynamic property, herein termed as FB-sp, which acts as a sperm stabiliser to be added to extensor media forming a mammal semen stabilising solution for mammals such as porcine, caprine, bovine, wild boars, horses, canine, felines, and even humans, preserving and conserving the germplasm of their gametes under refrigeration during the time between obtaining the semen sample from a breeding male and the time when this is used in artificial insemination (AI) methods.

### BACKGROUND OF THE INVENTION

Demand for animal meat (protein) and its derivatives increases annually, so livestock breeding farms must increase their production to meet said growing demand.

Artificial insemination (AI) method is the assisted reproduction technique most widely used by farms and/or producers of animals of commercial interest.

The current artificial insemination (AI) market provides nutritious liquid media, named sperm diluents or extenders, which can be added with bio-stabiliser molecules of different quality and price used to maintain and preserve sperm obtained from breeding male seminal fluid for longer periods of time.

AI technique requires collecting the male semen and adding preservation media containing a composition of micronutrients (extenders) that allow to obtain a higher spermatozoa survival, using these media the samples are maintained and protected under controlled cold, in the case of porcine sperm, for example, samples are kept between 15°C-17°C before being used in the process of female artificially assisted insemination.

Thus, the animal breeding industry needs molecular components as additive in sperm diluents or extenders, which have the functional property of stabilising sperm viability in order to strengthen the sperm fertilizing potential in AI process, either used for a single or a backup (second dose) insemination dose in order to improve the "fertilizing potential" conditions of the spermatozoa in the development of effective doses for AI to enhance the number of live births.

Diluents or extenders have been classified into two large groups, those whose objective is short-term conservation (less than 3 days), and those whose objective is long-term conservation (from 3 a 5 days). The former are mainly used in structures for short-term distribution of seminal doses, typical of European or Latin American systems, where the production of seminal doses in the same farm is frequent. While the long-term are typical of structures such as those present in the United States of America or Norway, where the distance between the place of seminal obtainment and production and the place to be used, i.e. female livestock breeding farms, is long. The advantages of long-term diluents are the possibility of preserving sperm viability over time for long-distance transport.

Methods of quality control and sperm safety are associated with semen diagnostic tests which are performed before the sperm is used, such as tests using PCR (Polymerase Chain Reaction) techniques to detect the presence of several viruses or complete analysis of seminal quality, whereby task organization in seminal collection farms is better organized and makes much easier sample distribution to breeding farms. However, long-term media have costs that make their massive and routine use impractical for livestock breeding farms in the intensive animal farming, in addition to requiring an antibiotic overload to ensure maintaining sample safety.

Xenobiotic factors are induced or produced during semen manipulation, such as thermal and biochemical changes that cause oxidative stress, which affect spermatozoa by damaging cell membranes, inducing an acrosomal reaction, decreasing their fertilizing potential and viability. This causes significant economic losses because the number of fertilized gametes is reduced, and thus the number of live births and consequently the animal production process decreases.

For preventing the induction of oxidative stress in concentrated ejaculated semen, the market has generated products such as liquid media known as seminal fluid diluents or extenders, which allow to preserve sperm cell functional characteristics and maintain the fertility level in this medium, artificially increasing the diluted volume of ejaculate. Media (extenders) available on the market, which are supplied to maintain the high metabolic activity necessary for sperm transport through the female genital tract, have not yet been able to solve the problem of short- and long-term spermatozoa preservation until their use in animal AI and, additionally, to maintain and preserve the germplasm, metabolic and biochemical capacity provided by male testicular seminal plasma, since they are affected by dilution (osmotic shock) inducing a reduction of the spermatozoa's reproductive efficiency.

The breeding males are classified as good or bad breeders, so that their selection is vital for obtaining breeders that provide semen having a maximum fertilizing potential "Premium males", which is critical for an intensive livestock farms. In order to preserve spermatozoa for extended periods, it is necessary to modulate the sperm metabolic activity by reducing temperature, so one of the functional objectives of sperm diluents is to refrigerate and preserve ejaculates having high phenotypic quality features to allow the progeny to inherit meat quality and the genotypic features of the selected male breeders belonging to Premium category, which are identified by having high rates of cell viability, sperm motility, low morphological aberration content and high fertilization rate at reproductive level.

Traditional diluents or extenders have a capacity for preserving spermatozoa's shell life from 5 to 7 days, and are designed for balanced osmotic levels and pH, at values from 7 to 8. However, their bioprotective capacity is still relatively low since they show a viability drop greater than 40% in the first 24 hours. Long-term diluents allow preserve spermatozoa with viability rates between 40 and 65% while short-term diluents reach only up to 45% at third day. These products would be useful for transporting the sample from livestock breeding farms to other locations, keeping viability for several hours after obtaining the ejaculate, by keeping the storage temperature at 15°C, however, this is expensive.

The advantage of adding the FB-sp described in the present invention is to obtain a low-cost extender, which manages to maintain semen fertility and prolificacy better than commercial extenders without additives, achieving a survival of over 65% after 7 days, being useful for artificial insemination with fresh chilled spermatozoa or also extending its biochemical potential in the management of sperm cryopreservation at temperatures greater than -180°C.

WO2009043128 describes the effect proteases isolated from Bauhinia sp. for the treatment of microbial infections and pharmaceutical composition of a native sequence of more than 250 amino acids corresponding to an antibiotic product with bactericidal activity. On the contrary, the present invention focuses on a cell membrane stabiliser that uses absolutely different biological mechanisms.

In this context, the present invention discloses a polypeptide bio-protector spermatic stabiliser (FB-sp), which is an inexpensive biotechnology polypeptide molecular alternative, (short chain having 10 amino acids) for supplementation of spermatic diluents, allowing to extend the sample shell life while maintaining seminal quality parameters, for example, viability, metabolism, progressive sperm motility, fertilizing potential, ability to reduce oxidizing agents without interfering with the sperm physiology and metabolic capacity. This represents a greater impact on the reproductive and economic potential to massify any animal species, for example the massification of AI Premium males samples in farms distant from the origin sample sites or transnational shipments for genetic improvement.

### BRIEF DESCRIPTION OF THE FIGURES

Fig. 1: Figure 1 shows Progressive Gametes viability (%) vs. Time (hours) (· = Using FB-sp bio-stabiliser ; ■ = without FB-sp bio-stabiliser), it shows percentage of pig spermatozoa with normal mobility suitable for fertilization, incubated in the presence of FB-sp 10 µM at 17°C.
Fig. 2: Concentration (ug/mL) of FB-sp in Plasma bbki4 vs. Time (hours). It shows plasma FB-sp concentration curve used in a liquid medium for a 90 kg animal.

### DETAILED DESCRIPTION OF THE INVENTION

The present invention describes a spermatic stabiliser, herein named FB-sp having an amino acid sequence, using a three letter nomenclature description, corresponding to: SEQ ID NO 1,
Arg, Pro, Gly, Leu, Pro, Val, Phe, Ser, Pro, Leu,
also written as one letter nomenclature:
RPGLPVFSPL,

An embodiment of the present invention is the use of said FB-sp additive to preserve male mammalian gametes, as well as the use of said additive to prepare a mammalian gamete bio-stabiliser composition for mammals such as porcine, caprine, bovine, wild boars, horses, canine, felines, or humans.

Other embodiment of the present invention is a bio-stabiliser composition comprising said FB-sp spermatic stabiliser used as a lyophilized product, at a concentration of up to 10µM (10 micromol), diluted in any extender medium available in the state of the art, the concentration of the FB-sp may increase up to 10% for treating low-ratio living cells.

Other embodiment is the previous bio-stabiliser composition optionally added with any cryopreservant available in the state of the art.

Other embodiment of the present invention consists of a process for preserving mammalian semen comprising the steps of:
a) Separating by centrifugation, at a value from 35 to 45 g for 5 to 15 minutes, the cell fraction (spermatozoa) of the semen obtained by manipulation of breeding males from the liquid fraction consisting of nutrient liquid medium, which allows settling and separating living cells from inert cell debris that is removed from the process;
b) Diluting the living cells in physiological medium, wherein short- or long-term extenders are added to form a bio-stabiliser composition;
c) Adding the sperm stabiliser FB-sp of sequence SEQ ID NO: 1 at a concentration of up to 10uM to the bio-stabiliser composition of step b) to form a fertilizing solution;
d) Keeping the fertilizer solution of stage c) in a cryopreservant medium at a temperature in the range from -180°C to 17°C, stored in an insulated capped flask, without contact with the environment until use, the fertilizing solution can be stored or transported under these conditions, optionally the temperature range is from 1°C to 17°C;
e) Transporting the fertilizing solution to the place *in situ* and raising its temperature to 17°C at the time of use and applying the fertilizing solution in the breeding females according to the artificial insemination protocols known in the state of the art;
f) Optionally in step c) a reactive oxygen species (ROS) stabilising complement is additionally added to the fertilizing solution when the living cell ratio is very small (<20% relative to the average pattern obtained for boards of the porcine farm). The stabiliser supplement may be the same FB-sp spermatic stabiliser increased by 10% in concentration, or the addition of traditional commercial antioxidant molecules such as albumins and others. The concentration will depend on the indications of each antioxidant molecule, considering that xenobiotic molecules that induce a hyperosmolarity shock (greater than 300mOSM) should never be added;

### APPLICATION EXAMPLE

### EXAMPLE 1.

A test of the progressive motility of gametes incubated in fertilizing solution added with the FB-sp bio-stabiliser polypeptide was performed. Progressive gametes are spermatozoa that have normal mobility capacity and are suitable for fertilizing.

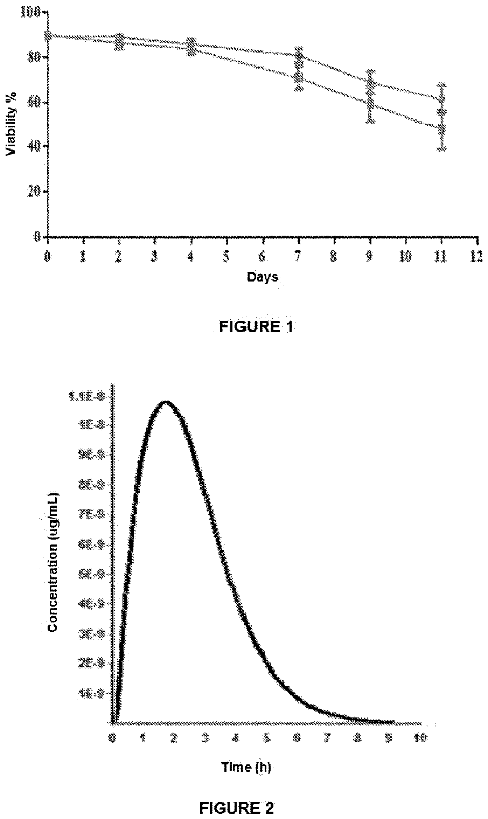

Figure 1 shows the Progressive Gametes viability (%) percentage vs. Time (hours) (• = using FB-sp bio-stabiliser; ■ = without FB-sp bio-stabiliser)

The results show the protective effect of FB-sp on the progressive motility of porcine spermatozoa stored for 72 h at 17°C. It can be seen that the incubation of spermatozoa of porcine boars with FB-sp (•) (10 µM) preserves progressive motility up to 85% of spermatozoa for a period of 7 days, while the control (■) without stabiliser decreases to a value of 70% on day 7, the difference increases over the days. These results are measured through optical microscopy, as measured by the CASA method for pig spermatozoa evaluation. This result is highly significant in relation to the values obtained when only spermatozoa incubation physiological media known in the art are used, such as MR-A^{®} or Androstar^{®}.

It is thus found that the FB-sp bio-stabiliser polypeptide provides a spermatozoa protective effect by extending their viability.

An analysis *"in silico"* made using GastroPlus simulation software provide some features of the FB-sp bio-stabiliser polypeptide: 1279 Da molecular weight, the structure of the 10 amino acids of FB-sp shows that only the conservative amino acid V (valine) and R (arginine) have conditions of active sites, i.e., the external pole Ac- has a negative charge and the external pole -NH2 (terminal amino acid), is positive, which determines that it is a hydrophilic and polar structure.

It was also seen that for a dose of 1 mg/mL, the absorption coefficient is 0.079 and the physicochemical dissolution coefficient in aqueous medium is 9.93×10³, depending on the calibration of the equipment in ionic liquid media used as controls, MR-A^{®} medium was used in this case. This means that it has a low absorption capacity in biological membranes such as spermatozoa membranes, vaginal mucosa membranes, intrauterine mucosa membrane, but it has a very good dissolution capacity in physiological medium thus forming an extensor solution.

Figure 2 shows the plasma concentration curve free of product FB-sp (µg/mL) administered in a liquid medium. When simulating the process for an animal weighing 90 kg, it can be seen that its blood plasma, after using the fertilizing solution in a concentration of 1 mg/mL of FB-sp diluted in MR-A^{®} nutrient medium (nutritive medium for the preservation of porcine semen), has concentrations after 8 hours on the order of only 1.1 × 10⁻⁸ µg/mL in plasma and that its maximum pick is obtained after 2 hours. Then, the *"in silico"* model shows that it has a very low permeability coefficient and low residual effect because it is fully degraded from the organism within the first 8 hours.

Demonstrating that the applied concentration does not have a cytotoxicity risk.

## Claims

1. A spermatic bio-stabiliser polypeptide FB-sp, **CHARACTERIZED in that** it has the amino acid sequence according to SEQ ID NO: 1 (using 1 letter nomenclature):
RPGLPVFSPL.

2. Use of the stabilising polypeptide of claim 1, **CHARACTERIZED in that** it serves to preserve mammalian gametes.

3. Use of the stabilising polypeptide of claim 1, **CHARACTERIZED in that** it serves to prepare a bio-stabiliser composition to preserve mammalian gametes.

4. The use of any of claims 2 or 3, **CHARACTERIZED in that** mammals are selected from porcine, caprine, bovine, wild boars, horses, canine, felines.

5. The use of any of claims 2 or 3, **CHARACTERIZED in that** the mammal is human.

6. A sperm bio-stabiliser composition, **CHARACTERIZED in that** it comprises:
the FB-sp spermatic bio-stabiliser of claim 1 in a concentration of up to 10 µM diluted in an extender medium.

7. The composition of claim 6, **CHARACTERIZED in that** it comprises a cryopreservant medium.

8. A process to stabilise animal semen, **CHARACTERIZED in that** the semen obtained from the ejaculate undergoes a manipulation process that includes the following stages:
a) separating the cell fraction (sperm) of the semen, obtained by manipulation of the breeding males, from the liquid fraction consisting of nutrient liquid medium;
b) diluting the living cells in physiological medium, wherein short- or long-term extenders are added to form a bio-stabiliser composition;
c) adding the FB-sp spermatic stabiliser of claim 1, in the bio-stabiliser composition obtained in step b), in a concentration of up to 10µM, to form a fertilizing solution;
d) keeping the fertilizing solution obtained in step c) in a cryopreserved or cold medium, at a temperature in the range from -180°C to 17°C, stored in an insulated capped flask without contact with the environment until use; the fertilizing solution can be stored or transported under these conditions.

9. The process according to claim 8, **CHARACTERIZED in that** in step d), the temperature range is between 1°C and 17°C.

10. The process according to claim 8, **CHARACTERIZED in that** in step a), the breeding males are selected from porcine, caprine, bovine, wild boars, horses, canine, felines.

11. The process according to claim 8, **CHARACTERIZED in that** in step a), the breeding males are human.

12. The process according to claim 8, **CHARACTERIZED in that** in step c), a reactive oxygen species (ROS) stabilising complement at a concentration of from 0.001-100 µM is added to the fertilizing solution.

## Patentansprüche

1. Spermienbiostabilisator-Polypeptid FB-sp, **dadurch GEKENNZEICHNET, dass** es die folgende Aminosäuresequenz gemäß SEQ ID NO: 1 (unter Verwendung der 1-Buchstaben-Nomenklatur) aufweist: RPGLPVFSPL.

2. Verwendung des Stabilisator-Polypeptids nach Anspruch 1, **dadurch GEKENNZEICHNET, dass** es zur Konservierung von Säugergameten dient.

3. Verwendung des Stabilisator-Polypeptids nach Anspruch 1, **dadurch GEKENNZEICHNET, dass** es zur Herstellung einer Biostabilisator-Zusammensetzung zur Konservierung von Säugergameten dient.

4. Verwendung nach einem der Ansprüche 2 oder 3, **dadurch GEKENNZEICHNET, dass** die Säuger ausgewählt sind aus Schweinen, Ziegen, Rindern, Wildschweinen, Pferden, Hunden und Katzen.

5. Verwendung nach einem der Ansprüche 2 oder 3, **dadurch GEKENNZEICHNET, dass** der Säuger menschlicher Natur ist.

6. Spermienbiostabilisator-Zusammensetzung, **dadurch GEKENNZEICHNET, dass** sie Folgendes enthält:
den FB-sp Spermienbiostabilisator nach Anspruch 1 in einer Konzentration von bis zu 10 µM, verdünnt in einem Streckmedium.

7. Zusammensetzung nach Anspruch 6, **dadurch GEKENNZEICHNET, dass** sie ein Kryokonservierungsmedium enthält.

8. Verfahren zur Stabilisierung von Tiersperma, **dadurch GEKENNZEICHNET, dass** das aus dem Ejakulat gewonnene Sperma einem Bearbeitungsverfahren unterzogen wird, das die folgenden Schritte umfasst:
a) Abtrennung der Zellfraktion (Spermien) des durch Manipulation der männlichen Zuchttiere gewonnenen Samens von der aus flüssigem Nährmedium bestehenden flüssigen Fraktion;
b) Verdünnen der lebenden Zellen in einem physiologischen Medium, dem zur Bildung einer Biostabilisator-Zusammensetzung Kurzzeit- oder Langzeitstreckmittel zugesetzt werden;
c) Zugabe des FB-sp Spermienstabilisators nach Anspruch 1 zu der in Schritt b) gewonnenen Biostabilisator-Zusammensetzung in einer Konzentration von bis zu 10µM zur Herstellung einer Befruchtungslösung;
d) Aufbewahrung der in Schritt c) gewonnenen Befruchtungslösung in einem kryokonservierten oder kalten Medium bei einer Temperatur im Bereich von -180 °C bis 17 °C, gelagert in einer isolierten Flasche mit Deckel ohne Umgebungskontakt bis zur Verwendung; wobei die Befruchtungslösung unter diesen Bedingungen gelagert oder transportiert werden kann.

9. Verfahren nach Anspruch 8, **dadurch GEKENNZEICHNET, dass** der Temperaturbereich in Schritt d) zwischen 1 °C und 17 °C liegt.

10. Verfahren nach Anspruch 8, **dadurch GEKENNZEICHNET, dass** die männlichen Zuchttiere in Schritt a) ausgewählt sind aus Schweinen, Ziegen, Rindern, Wildschweinen, Pferden, Hunden und Katzen.

11. Verfahren nach Anspruch 8, **dadurch GEKENNZEICHNET, dass** die männlichen Zuchttiere in Schritt a) menschlicher Natur sind.

12. Verfahren nach Anspruch 8, **dadurch GEKENNZEICHNET, dass** der Befruchtungslösung in Schritt c) ein die reaktive Sauerstoffspezies (ROS) stabilisierendes Komplement in einer Konzentration von 0,001-100 µM zugesetzt wird.

## Revendications

1. Bio-stabilisateur spermatique polypeptide FB-sp, **CARACTÉRISÉ en ce qu'**il a la séquence d'acides aminés selon SEQ ID NO : 1 (en utilisant la nomenclature à 1 lettre) : RPGLPVFSPL.

2. Utilisation du stabilisant polypeptide de la revendication 1, **CARACTÉRISÉE en ce qu**'elle sert à préserver les gamètes de mammifères.

3. Utilisation du stabilisant polypeptide de la revendication 1, **CARACTÉRISÉE en ce qu'**elle sert à préparer une composition bio-stabilisante pour préserver les gamètes de mammifères.

4. Utilisation de l'une quelconque des revendications 2 ou 3, **CARACTÉRISÉE en ce que** les mammifères sont choisis parmi les porcins, les caprins, les bovins, les sangliers, les chevaux, les canins, les félins.

5. Utilisation de l'une quelconque des revendications 2 ou 3, **CARACTÉRISÉE en ce que** le mammifère est humain.

6. Composition de bio-stabilisateur spermatique, **CARACTÉRISÉE en ce qu**'elle comprend :
le bio-stabilisateur spermatique FB de la revendication 1 à une concentration allant jusqu'à 10 µM dilué dans un milieu diluant.

7. La composition de la revendication 6, **CARACTÉRISÉE en ce qu'**elle comprend un milieu cryoconservateur.

8. Procédé de stabilisation de sperme animal, **CARACTÉRISÉ en ce que** le sperme obtenu à partir de l'éjaculat subit un processus de manipulation qui comprend les étapes suivantes :
a) séparation de la fraction cellulaire (sperme) du sperme, obtenue par manipulation des mâles reproducteurs, de la fraction liquide constituée d'un milieu liquide nutritif ;
b) dilution des cellules vivantes dans un milieu physiologique, dans lequel sont ajoutés des extenseurs à court ou à long terme pour former une composition bio-stabilisante ;
c) ajouter le stabilisateur spermatique FB de la revendication 1, dans la composition de bio-stabilisateur obtenue à l'étape b), à une concentration allant jusqu'à 10µM, pour former une solution de fertilisation ;
d) conserver la solution fertilisante obtenue à l'étape c) dans un milieu cryoconservé ou froid, à une température comprise entre -180°C et 17°C, stockée dans un flacon capsulé isolé sans contact avec l'environnement jusqu'à son utilisation ; la solution fertilisante peut être stockée ou transportée dans ces conditions.

9. Procédé selon la revendication 8, **CARACTÉRISÉ en ce que** dans l'étape d), la plage de température est comprise entre 1°C et 17°C.

10. Procédé selon la revendication 8, **CARACTÉRISÉ en ce que** dans l'étape a), les mâles reproducteurs sont choisis parmi les porcins, les caprins, les bovins, les sangliers, les chevaux, les canins, les félins.

11. Procédé selon la revendication 8, **CARACTÉRISÉ en ce que** dans l'étape a), les mâles reproducteurs sont humains.

12. Le procédé selon la revendication 8, **CARACTÉRISÉ en ce que** dans l'étape c), un complément stabilisant les espèces réactives de l'oxygène (ROS) à une concentration de 0,001 à 100 µM est ajouté à la solution fertilisante.
